# EUROPEAN PATENT SPECIFICATION

(11) **EP 3 222 234 B1**
(45) Date of publication and mention of the grant of the patent: **21.10.2020**
(21) Application number: 16858774.9
(22) Date of filing: 26.07.2016
(51) Int. Cl.: A61B 17/86, A61L 31/08, A61B 17/74

(54) **POROUS TANTALUM METAL HOLLOW SCREW**
HOHLSCHRAUBE AUS PORÖSEM TANTALMETALL
VIS CREUSE POREUSE EN MÉTAL DE TANTALE

(30) Priority: 28.10.2015 CN 201510712216; 28.10.2015 CN 201520845079 U
(43) Date of publication of application: 27.09.2017
(73) Proprietor: Zhao, Dewei, Dalian, Liaoning 116001 (CN); Wang, Benjie, Dalian, Liaoning 116001 (CN); Xie, Hui, Dalian, Liaoning 116001 (CN); Huang, Shibo, Dalian, Liaoning 116001 (CN); Yu, Xiaowei, Dalian, Liaoning 116001 (CN); Wang, Wei, Dalian, Liaoning 116001 (CN)
(72) Inventor: Zhao, Dewei, Dalian, Liaoning 116001 (CN); Wang, Benjie, Dalian, Liaoning 116001 (CN); Xie, Hui, Dalian, Liaoning 116001 (CN); Huang, Shibo, Dalian, Liaoning 116001 (CN); Yu, Xiaowei, Dalian, Liaoning 116001 (CN); Wang, Wei, Dalian, Liaoning 116001 (CN)
(74) Representative: Proi World Intellectual Property GmbH
(86) International application number: PCT/CN2016/091730
(87) International publication number: WO 2017/071335

(56) References cited:
- WO-A2-01/62136
- CN-A- 1 269 198
- CN-A- 103 083 074
- CN-A- 103 462 730
- CN-A- 105 147 383
- CN-B- 103 462 730
- CN-U- 202 069 679
- CN-U- 203 328 868
- CN-U- 203 988 332
- CN-U- 204 428 147
- CN-U- 205 083 596
- JP-A- H08 238 265
- US-A1- 2008 262 556
- US-A1- 2011 093 020

## Description

### Technical Field

The present invention relates to a medical permanent implant, particularly to a porous tantalum metal hollow screw of medical implant for treatment of femoral neck fracture.

### Background Art

Femoral neck fracture is a common clinical disease. There also exist some debates on the treatment of the femoral neck fracture. Most scholars believe that fresh femoral neck fractures should be treated following a principle of striving to treat early, conducting anatomical reduction and being fixed firmly; however, current internal fixation methods are basically fixed using hollow compression screws. At present, hollow screws made of titanium are adopted clinically. Although certain clinical results have been achieved so far, but it has clinical defects such as no osteogenesis induction and needs secondary removal.

Porous medical metal implant materials have important and special purposes of treating bone tissue trauma and inducing bone tissue regeneration, these materials usually are porous stainless steel, porous titanium and so on. As a porous implant material used for the treatment of bone tissue trauma and defect, a porosity of the material should reach 30-85%, and pores are preferably all communicated and uniformly distributed, or the pores are partially communicated and uniformly distributed according to the need, so as to be consistent with the bone tissue growth of the human body, as well as to reduce the weight of the material itself to fit the human body implantation.

Metal tantalum has no toxic side effects on human body, and has good biocompatibility and mechanical properties. The porous material of the metal tantalum is expected to replace the traditional medical metal biomaterials and become the main biomaterials in the field of the treatment of femoral neck fracture. With the rapid development of medical science at home and abroad and the further understanding of tantalum as a human body implant material, the demands of people on the porous metal tantalum material of the human body implantation become more and more urgent, and the requirements on the porous metal tantalum material are also increasing. As porous medical implant metal tantalum, if it can have a high uniformly distributed and communicated pores, and physical and mechanical properties to adapt to the human body, it is an important material of connecting piece to ensure the normal growth of new bone tissue.

For the materials porous tantalum, US5282861 discloses an open cell tantalum material for cancellous bone implants and cell and tissue receptors and preparation thereof. This porous tantalum is made of commercial pure tantalum, and a carbon skeleton obtained by thermal degradation of polyurethane precursor is served as support. The carbon skeleton is a multi-dodecahedron in which a grid-like structure exists. The carbon skeleton is entirely distributed with micropores, and has a porosity as high as 98%, and then the commercial pure tantalum is bonded to the carbon skeleton by a chemical vapor deposition and penetrative method to form a porous metal microstructure, which is called a chemical deposition method for short. A thickness of a tantalum layer on the surface of the porous tantalum material obtained by this method is 40-60µm; and the specific gravity of the tantalum is about 99% and that of the carbon skeleton is about 1% in the entire porous material. It is further documented that the porous material has a compressive strength of 50-70MPa, an elastic modulus of 2.5-3.5GPa, a tensile strength of 63MPa, and a plastic deformation of 15%.

US20110093020 A1 discloses a poly-porous (micropore) hollow screws as diffusion chamber filled with core matrix for targeted delivery of growth factors and bone marrow stem cells. The screws comprise at least two parts: the distal part of the screw consists of the tip of the screw made of poly porous material and hollow inside proximally. Assembly of screw created a chamber in the middle of the screw. The chamber is filled with core matrix consisting of gelatin nanoparticles pre-impregnated with material for bone or for tendon and fibrin sealants or Chitosan dispersed with bone marrow stem cells and/or other growth factors. Once the screw implanted in the human body, at the mean time as the bone regenerate and/or in growth, mechanical strength of the screw increased.

At present, the porous tantalum metal hollow screws of medical implants made of
the current porous tantalum materials for the treatment of femoral neck fractures have poor biomechanics and biocompatibility, so how to improve their mechanical strength and biological compatibility is an important subject.

The hollow compression screws currently used in the clinical fixation of femoral neck fractures have a variety of structures, for example, the hollow screws made of metal titanium or the like have a shorter triangular thread structure, and most of tail nail heads of the hollow screws made of other materials have circular structure. These screws exist many common clinical problems, such as complex structure, poor biomechanical performance, insufficient mechanical strength and needing secondary removal. Problems, such as internal fixation screw cracking and fracture re-displacement, often occur in the application.

### Summary of the Invention

Aiming at the problems of the existing hollow compression screws, an object of the present invention is to provide a porous tantalum metal hollow screw for treatment of femoral neck fracture. The porous tantalum metal hollow screw has the advantages of simple structure, preferable mechanical strength and biological performance, firm internal fixation performance, higher tissue compatibility and biomechanical properties, and can induce bone tissue formation and facilitate bone tissue ingrowth.

The technical solutions of the present invention are as follows:
A porous tantalum metal hollow screw, comprising a screw shaft and a screw head, the screw shaft and the screw head are integrally formed, a thread is disposed on outer surface of the front end of the screw shaft, wherein the ratio of the length of thread to the length of the screw shaft is 1:1.7-2.3, the screw head is a conical screw head having a cylindrical screw top, an included angle between a conical surface of the conical screw head and a central line of the screw shaft ranges from 44° to 46°, the outer surfaces of the two sides of the cylindrical screw top are provided with grooves which are symmetrically arranged on the central line of the screw shaft, the included angle between the two inclined surfaces of each groove is 60°, a through hole is formed in the central shaft of the screw shaft, and a sliding shaft is disposed between the thread and the screw head.

Further, wherein a pitch of the thread is 3.7-4.3mm, preferably 4mm; a width of the thread is 1.7-2.3mm, preferably 2mm; the difference between an outer diameter and an inner diameter of the thread is 7-13% of diameter of the screw shaft, preferably 10%; and a helix angle of the thread is 90±2°; the above design of the thread is beneficial to reducing the resistance in the screwing-in process of the screw, and reducing the step of self-tapping by other equipment before the traditional screw is screwed in, and the deepening of the thread is beneficial to the rigid fixation of the fracture regions.

Further, wherein a depth of the grooves is 7-13% of the diameter of the screw shaft, preferably 10%.

Further, wherein a thickness of the screw head is 7-13% of the length of the screw shaft, preferably 10%. Such design strengthens a holding force at the tail of the hollow screw, so that the tail of the hollow screw is firmly fixed in bone cortex portion to prevent screw retrogression and fracture displacement.

Further, the thread is a triangular shape. Such design reduces the self-tapping process before the screw is screwed in, reduces the bone loss, and strengthens the internal fixation strength at the bone fracture part.

Further, wherein a flank angle of the thread is 30°-50°. Such design is beneficial to the fixation of the screw.

Further, wherein a diameter of the through hole is 77-83% of the diameter of the screw shaft, preferably 80%.

Further, wherein a section of the screw shaft far away from the screw head is the front end of the screw, a vertex angle of the front end of the screw is in a conical shape of 30-120°.

Further, wherein a material adopted by the hollow screw is prepared by method of chemical vapor deposition, which reducing tantalum metal compound to tantalum metal powder, and uniformly depositing the tantalum metal powder on a surface of a graphite carbon skeleton to form tantalum coating; wherein the tantalum metal compound is one of tantalum chloride and tantalum fluoride; a porosity of the graphite carbon skeleton is greater than 85%, a pore diameter is 200-600µm; and a thickness of the tantalum coating is 40-60µm.

The above material used for preparing the porous tantalum metal hollow screw has a porosity of 85% and a pore diameter of 200-600µm, and the material is in a reticular structure with at least 80% mutually interlaced polyhedron and has dodecahedron reticular structure with three-dimensional connectivity distribution of pores, which is conducive bone cell and tissue ingrowth.

The present invention further provides a preparation method for the porous tantalum metal hollow screw. The preparation method comprises the following steps of:
(1) etching the graphite carbon skeleton with dilute hydrochloric acid for 10-20min, then washing the graphite carbon skeleton with water and ethanol in turn, and then blowing it with nitrogen to dry, and placing the graphite carbon skeleton into a reaction chamber;
(2) simultaneously providing a processing gas and hydrogen into the reaction chamber to react for 4-6 h at 900-1050°C under a vacuum degree of 10-15Pa;
wherein, the processing gas comprises the tantalum metal compound and carrier gas, the tantalum metal compound is put into a source tank and heated to 120-250°C, and an inert gas at 300°C as the carrier gas is provided into the reaction chamber;
wherein the inert gas is one of argon and helium, or a mixture of argon and helium;
a flow rate of the processing gas is 80-100ml /min; and
a flow rate of the hydrogen is 100-150 ml/min.

The porous tantalum metal hollow screw of the present invention can be applied to medical implants for treating nonunion, bone defect and prosthesis reconstruction after tumor resection.

The present invention has the advantageous effects that:
1. The thread of the front of the screw of the present invention is longer than that of the traditional hollow screw, a screw head with a cylindrical screw top is adopted, and rectangular grooves having a specific included angle (60°) is arranged on the cylindrical screw top. Through such design, screwing-in and firm fixation of the hollow screw are facilitated, secondary displacement at a bone fracture part is avoided, and the defects, that the square or round screw head and the design of profiles and counter bore directly arranged on the screw head in the prior art cannot fit the cortical bone and cannot fixed firmly in the using process, are overcome well;
2. The screw of the prevent invention can avoid the defect of the tail part deformation when the screw is screwed in, so that a force reaches the front end uniformly, and the possibility of the screw deformation and cracking is reduced; the screw head has a compression effect, and has a better holding and fixing effects; the deformation and cracking of the thread is avoided when implanting the cortical bone and other hard sclerotin; and the screw does not need to be removed through second operation, so as to lighten the suffering of patients.

The porous tantalum metal hollow screw of the present invention is beneficial to the formation of new bone and replacement of the bone, especially has better clinical effect in the treatment of femoral neck fracture, and overcomes the insufficient effect of the existing titanium alloy "hollow screw" in clinical treatment. On the other hand, the material of the porous tantalum metal hollow screw of the present invention has a dodecahedron three-dimensional reticular structure, which is similar to that of trabeculae of cancellous bone, and has high histocompatibility, can induce bone tissue formation, and facilitates bone tissue ingrowth; the material also has strong biomechanical properties, can provide mechanical support, so as to achieve the purposes of inducing and replacing the bone tissues. The porous tantalum metal hollow screw of the present invention has the advantages of simple preparation method, low cost and favorable implementation and clinical application, and is suitable for the needs of the majority of patients.

### Brief Description of the Drawings

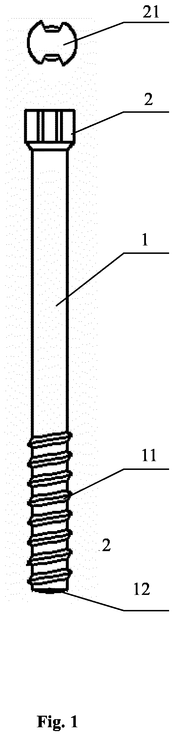

Fig. 1 is a schematic structural diagram of a hollow screw of the present invention.

Marks in figure: 1 refers to screw shaft, 11 refers to thread, 12 refers to the front end of screw, 2 refers to screw head, and 21 refers to groove.

### Detailed Description of the Preferred Embodiments

The following nonrestrictive embodiments can enable those ordinary persons skilled in the art to comprehensively understand the present invention, without limiting the present invention in any way.

### Embodiment 1

As shown in Fig. 1, a porous tantalum metal hollow screw comprises a screw shaft 1 and a screw head 2. The screw shaft 1 and the screw head 2 are integrally formed, and a thread 11 is disposed on the outer surface of the front end of the screw shaft 1; wherein the ratio of the length of the thread 11 to the length of the screw shaft 1 is 1:2. The screw head 2 is a conical screw head having a cylindrical screw top, the included angle between the conical surface of the conical screw head and the central line of the screw shaft is 45°, grooves 21 which are symmetric about the central line of the screw shaft 1 are formed in the outer surfaces of the two sides of the cylindrical screw top, the included angle between the two inclined surfaces of each groove 21 is 60°, a through hole is formed in the central shaft of the screw shaft 1, and the sliding shaft between the thread 11 and the screw head 2 is smooth;
wherein:
a pitch of the thread 11 is 4mm, a width of the thread 11 is 2mm, the difference between an outer diameter and an inner diameter of the thread 11 is 10% of a diameter of the screw shaft 1, and a helix angle of the thread is 91°. Such design of the thread 11 is beneficial to reducing the self-tapping process while the screw is screwed in and deepening of the thread 11, so as to beneficial to the rigid fixation of the fracture regions.

A depth of the grooves 21 is 10% of the diameter of the screw shaft 1.

A thickness of the screw head 2 is 10% of a length of the screw shaft 1; such design strengthens a holding force at the tail of the hollow screw, so that the tail of the hollow screw is firmly fixed in bone cortex portion to prevent screw retrogression and fracture displacement.

The thread 11 is in a triangular shape. Such design reduces the self-tapping process before the screw is screwed in, reduces the bone loss, and strengthens the internal fixation strength at the bone fracture part.

A flank angle of the thread 11 is 35°. Such design is beneficial to the fixation of the screw.

The diameter of the through hole is 80% of the diameter of the screw shaft 1.

A section of the screw shaft 1 far away from the screw head 2 is the front end 12 of the screw, a vertex angle of the front end 12 of the screw is in a conical shape of 120°, and a length of the front end of the screw is 3.3% of the length of the screw shaft.

The specific size of the screw can be appropriately adjusted according to the size, tissue or the like of the implanting position in specific use to achieve the best therapeutic effect.

### Embodiment 2

The porous tantalum metal hollow screw as described in embodiment 1 is prepared by the following method.
(1) The graphite carbon skeleton is etched with dilute hydrochloric acid for 10 min, and then the graphite carbon skeleton is washed with water and ethanol in turn, blown with nitrogen to dry, and placed into a reaction chamber.
(2) The tantalum chloride powder with a diameter of 400-mesh is put into a source tank and heated to 150°C, then provided into a reaction chamber with high temperature argon (300°C) as a carrier gas. The flow rate of the carrier gas is 100ml/min, the temperature of the reaction chamber is 1050°C, and the vacuum degree of the reaction chamber is 10Pa. Hydrogen is provided at a flow rate of 120ml/min while the tantalum chloride powder is provided into the reaction chamber using argon as the carrier gas, and the reduction reaction is carried out for 4 hours, so that the tantalum metal that reduced to metal powder is uniformly deposited on the surface of the graphite carbon skeleton and the surface of the pores to obtain the porous tantalum metal hollow screw of the present invention.

The porous tantalum metal hollow screw body material has a porosity of 85%, an elastic modulus of 10 GPa, and a maximum compressive strength of 35 MPa, wherein the porosity is detected according to the method in national standard GB/T 21650.1-2008, and the elastic modulus is detected according to the method in national standard GB/T 22315-2008.

The body material of the porous tantalum metal hollow screw has a dodecahedron reticular structure with three-dimensional connectivity distribution of pores, which is similar to that of trabeculae of cancellous bone, and has high histocompatibility, can induce bone tissue formation, and facilitates bone tissue ingrowth; the material also has strong biomechanical properties, can provide certain mechanical support, so as to achieve the purposes of promoting fracture healing and repair and filling the bone defects.

In the above-mentioned preparation method, the graphite carbon skeleton is first processed into the shape described in Fig. 1, and then the porous tantalum metal hollow screw of the present invention is prepared through steps (1) to (2).

## Claims

1. A porous tantalum metal hollow screw consisting of a material prepared by chemical vapor deposition, the metal hollow screw comprising a graphite carbon skeleton with a porosity of at least 85% and a pore diameter of 200-600µm and a tantalum coating formed by uniformly depositing a tantalum metal powder on the surface of the graphite carbon skeleton, the metal hollow screw comprising a screw shaft (1) and a screw head (2), wherein the screw shaft and the screw head are integrally formed, a thread (11) is disposed on outer surface of the front end of the screw shaft, wherein the ratio of the length of thread to the length of the screw shaft is 1:1.7-2.3, the screw head is a conical screw head having a cylindrical screw top, an included angle between a conical surface of the conical screw head and a central line of the screw shaft ranges from 44° to 46°, the outer surfaces of the two sides of the cylindrical screw top are provided with grooves (21) which are symmetrically arranged on the central line of the screw shaft, the included angle between the two inclined surfaces of each groove is 60°, a through hole is formed in the central shaft of the screw shaft, wherein a diameter of the through hole is 77-83% of the diameter of the screw shaft, and a sliding shaft is disposed between the thread and the screw head.

2. The porous tantalum metal hollow screw according to claim 1, wherein a pitch of the thread is 3.7-4.3mm, a width of the thread is 1.7-2.3mm, difference between an outer diameter and an inner diameter of the thread is 7-13% of diameter of the screw shaft, and a helix angle of the thread is 90±2°.

3. The porous tantalum metal hollow screw according to claim 1, wherein a depth of the grooves is 7-13% of the diameter of the screw shaft.

4. The porous tantalum metal hollow screw according to claim 1, wherein a thickness of the screw head is 7-13% of the length of the screw shaft.

5. The porous tantalum metal hollow screw according to claim 1, wherein the thread is a triangular shape.

6. The porous tantalum metal hollow screw according to claim 1, wherein a flank angle of the thread ranges from 30° to 50°.

7. The porous tantalum metal hollow screw according to claim 1, wherein a section of the screw shaft far away from the screw head is the front end of the screw, a vertex angle of the front end of the screw is in a conical shape of 30-120°.

8. The porous tantalum metal hollow screw according to claim 1, wherein a material adopted by the hollow screw is prepared by method of chemical vapor deposition, which reducing tantalum metal compound to tantalum metal powder, and uniformly depositing the tantalum metal powder on surface of a graphite carbon skeleton to form tantalum coating; wherein the tantalum metal compound is one of tantalum chloride and tantalum fluoride; a porosity of the graphite carbon skeleton is greater than 85%, pore diameter is 200-600µm; and a thickness of the tantalum coating is 40-60µm.

9. A preparation method for the porous tantalum metal hollow screw according to claim 8, comprising the following steps of:
(1) etching the graphite carbon skeleton according to claim 8 with dilute hydrochloric acid for 10-20min, then washing the graphite carbon skeleton with water and ethanol in turn, then blowing it with nitrogen to dry, and placing the graphite carbon skeleton into a reaction chamber;
(2) simultaneously providing a processing gas and hydrogen into the reaction chamber to react for 4-6h at 900-1050°C under a vacuum degree of 10-15Pa;
wherein: the processing gas comprises tantalum metal compound and carrier gas, the tantalum metal compound is put into a source tank and heated to 120-250°C, and an inert gas at 300°C as the carrier gas is provided into the reaction chamber;
wherein the inert gas is one of argon and helium, or a mixture of the two;
a flow rate of the processing gas is 80-100ml /min; and
a flow rate of the hydrogen is 100-150 ml/min.

## Patentansprüche

1. Poröse Tantalmetall-Hohlschraube, die aus einem durch chemische Dampfabscheidung hergestellten Material besteht, wobei die Metall-Hohlschraube ein Graphit-Kohlenstoff-Grundgerüst mit einer Porosität von mindestens 85% und einem Porendurchmesser von 200-600µm und eine Tantalbeschichtung aufweist, die durch gleichmäßiges Abscheiden eines Tantalmetallpulvers auf der Oberfläche des Graphit-Kohlenstoff-Grundgerüsts gebildet wird, wobei die Metall-Hohlschraube einen Schraubenschaft (1) und einen Schraubenkopf (2) aufweist, wobei der Schraubenschaft und der Schraubenkopf einstückig ausgebildet sind, ein Gewinde (11) an der Außenfläche des vorderen Endes des Schraubenschaftes angeordnet ist, wobei das Verhältnis der Länge des Gewindes zur Länge des Schraubenschaftes 1:1,7-2.3 beträgt, der Schraubenkopf ein konischer Schraubenkopf mit einem zylindrischen Schraubenkopf ist, ein eingeschlossener Winkel zwischen einer konischen Oberfläche des konischen Schraubenkopfes und einer Mittellinie des Schraubenschaftes im Bereich von 44° bis 46° liegt, die Aussenflächen der beiden Seiten des zylindrischen Schraubenkopfes mit Nuten (21) versehen sind, die symmetrisch auf der Mittellinie des Schraubenschaftes angeordnet sind, der eingeschlossene Winkel zwischen den beiden geneigten Flächen jeder Nut 60° beträgt, ein Durchgangsloch im zentralen Schaft des Schraubenschafts ausgebildet ist, wobei ein Durchmesser des Durchgangslochs 77-83% des Durchmessers des Schraubenschafts beträgt, und ein Gleitschaft zwischen dem Gewinde und dem Schraubenkopf angeordnet ist.

2. Poröse Tantalmetall-Hohlschraube nach Anspruch 1, bei der die Gewindesteigung 3,7-4,3 mm, die Gewindebreite 1,7-2,3 mm, der Unterschied zwischen dem Außendurchmesser und dem Innendurchmesser des Gewindes 7-13 % des Durchmessers des Schraubenschafts und der Steigungswinkel des Gewindes 90+/-2° betragen.

3. Poröse Tantalmetall-Hohlschraube nach Anspruch 1, wobei die Tiefe der Rillen 7-13% des Durchmessers des Schraubenschafts beträgt.

4. Poröse Tantal-Metall-Hohlschraube nach Anspruch 1, bei der die Dicke des Schraubenkopfes 7-13% der Länge des Schraubenschaftes beträgt.

5. Poröse Tantal-Metall-Hohlschraube nach Anspruch 1, wobei das Gewinde eine dreieckige Form hat.

6. Poröse Tantal-Metall-Hohlschraube nach Anspruch 1, wobei ein Flankenwinkel des Gewindes im Bereich von 30° bis 50° liegt.

7. Poröse Tantal-Metall-Hohlschraube nach Anspruch 1, bei der ein vom Schraubenkopf entfernter Abschnitt des Schraubenschaftes das vordere Ende der Schraube bildet, wobei ein Scheitelwinkel des vorderen Endes der Schraube eine konische Form von 30∼120° aufweist.

8. Poröse Tantalmetall-Hohlschraube nach Anspruch 1, bei der ein Material, woraus die Hohlschraube besteht, durch ein Verfahren der chemischen Dampfabscheidung hergestellt wird, bei dem eine Tantalmetallverbindung zu Tantalmetallpulver reduziert und das Tantalmetallpulver gleichmäßig auf der Oberfläche eines Graphit-Kohlenstoff-Gerüsts abgeschieden wird, um eine Tantalbeschichtung zu bilden; wobei die Tantalmetallverbindung eines von Tantalchlorid und Tantalfluorid ist; eine Porosität des Graphitkohlenstoffgerüsts größer als 85% ist, der Porendurchmesser 200-600µm beträgt; und die Dicke der Tantalbeschichtung 40-60µm beträgt.

9. Herstellungsverfahren für die poröse Tantalmetall-Hohlschraube nach Anspruch 8, umfassend die folgenden Schritte:
(1) Ätzen des Graphit-Kohlenstoffgerüsts nach Anspruch ' 8 mit verdünnter Salzsäure für 10-20min, dann Waschen des Graphit-Kohlenstoffgerüsts mit Wasser und Ethanol nacheinander, dann zum Trocknen Anblasen mit Stickstoff und Einbringen des Graphit-Kohlenstoffgerüsts in eine Reaktionskammer;
(2) gleichzeitiges Zuführen eines Prozessgases und von Wasserstoff in die Reaktionskammer, um 4-6h bei 900-1050°C unter einem Vakuumgrad von 10-15Pa zu reagieren;
wobei: das Prozeßgas eine Tantalmetallverbindung und ein Trägergas umfaßt, die Tantalmetallverbindung in einen Quelltank gegeben und auf 120-250°C erhitzt wird, und ein Inertgas bei 300°C als Trägergas in die Reaktionskammer eingebracht wird;
wobei das Inertgas eines von Argon und Helium oder eine Mischung der beiden ist; eine Strömungsgeschwindigkeit des Prozeßgases beträgt 80-100 ml/min; und eine Strömungsgeschwindigkeit des Wasserstoffs beträgt 100-150 ml/min.

## Revendications

1. Une vis creuse poreuse en tantale métallique constituée d'un matériau préparé par dépôt chimique en phase vapeur, la vis creuse métallique comprenant un squelette de carbone graphite avec une porosité d'au moins 85% et un diamètre de pore de 200-600µm et un revêtement de tantale formé en déposant uniformément une poudre de tantale métallique sur la surface du squelette de carbone graphite, la vis creuse métallique comprenant une tige de vis (1) et une tête de vis (2), dans laquelle la tige de vis et la tête de vis sont formées d'un seul tenant, un filetage (11) est disposé sur la surface extérieure de l'extrémité avant de la tige de vis, dans lequel le rapport de la longueur du filetage à la longueur de la tige de vis est de 1:1.7-2.3, la tête de vis est une tête de vis conique ayant un sommet de vis cylindrique, un angle inclus entre une surface conique de la tête de vis conique et une ligne centrale de la tige de vis va de 44° à 46°, les surfaces extérieures des deux côtés du sommet de vis cylindrique sont pourvues de rainures (21) qui sont disposées symétriquement sur la ligne centrale de la tige de vis, l'angle inclus entre les deux surfaces inclinées de chaque rainure est de 60°, un trou traversant est formé dans la tige centrale de la tige de vis, dans lequel un diamètre du trou traversant est de 77-83% du diamètre de la tige de vis, et une tige coulissante est disposée entre le filetage et la tête de vis.

2. Vis creuse poreuse en tantale métallique selon la revendication 1, dans laquelle un pas du filet est de 3,7-4,3 mm, la largeur du filet est de 1,7-2,3 mm, la différence entre le diamètre extérieur et le diamètre intérieur du filet est de 7-13% du diamètre de la tige de la vis, et l'angle d'hélice du filet est de 90+/-2°.

3. Vis creuse poreuse en tantale métallique selon la revendication 1, dans laquelle la profondeur des rainures est de 7 à 13 % du diamètre de la tige de la vis.

4. Vis creuse poreuse en tantale métallique selon la revendication 1, dans laquelle l'épaisseur de la tête de vis est de 7 à 13 % de la longueur de la tige de vis.

5. Vis creuse poreuse en tantale métallique selon la revendication 1, dans laquelle le filetage est de forme triangulaire.

6. Vis creuse poreuse en tantale métallique selon la revendication 1, dans laquelle l'angle de flanc du filetage est compris entre 30° et 50°.

7. Vis creuse en tantale métallique poreux selon la revendication 1, dans laquelle une section de la tige de la vis éloignée de la tête de la vis est l'extrémité avant de la vis, un angle de sommet de l'extrémité avant de la vis est de forme conique de 30∼120°.

8. Vis creuse poreuse en tantale métallique selon la revendication 1, dans laquelle un matériau adopté par la vis creuse est préparé par une méthode de dépôt chimique en phase vapeur, qui réduit le composé de tantale métallique en poudre de tantale métallique, et qui dépose uniformément la poudre de tantale métallique sur la surface d'un squelette de carbone graphite pour former un revêtement de tantale ; dans lequel le composé de tantale métallique est l'un parmi le chlorure de tantale et le fluorure de tantale ; une porosité du squelette de carbone de graphite est supérieure à 85%, le diamètre des pores est de 200-600µm ; et une épaisseur du revêtement de tantale est de 40-60µm.

9. Procédé de préparation de la vis creuse poreuse en tantale métallique selon la revendication 8, comprenant les étapes suivantes
(1) Graver le squelette de carbone en graphite selon la revendication 8 avec de l'acide chlorhydrique dilué pendant 10-20 minutes, puis laver le squelette de carbone en graphite avec de l'eau et de l'éthanol à tour de rôle, puis le souffler avec de l'azote pour le sécher, et placer le squelette de carbone en graphite dans une chambre de réaction ;
(2) l'introduction simultanée d'un gaz de traitement et d'hydrogène dans la chambre de réaction pour qu'ils réagissent pendant 4-6h à 900-1050°C sous un degré de vide de 10-15Pa ; où : le gaz de traitement comprend un composé de tantale métallique et un gaz porteur, le composé de tantale métallique est placé dans un réservoir source et chauffé à 120-250°C, et un gaz inerte à 300°C en tant que gaz porteur est fourni dans la chambre de réaction ; dans lequel le gaz inerte est l'un parmi l'argon et l'hélium, ou un mélange des deux ; un débit du gaz de traitement est de 80-100ml/min ; et un débit de l'hydrogène est de 100-150ml/min.
